# EUROPEAN PATENT APPLICATION

(11) **EP 1 314 399 A2**
(43) Date of publication of application: **28.05.2003**
(21) Application number: 02026078.2
(22) Date of filing: 22.11.2002
(51) Int. Cl.: A61B 17/34

(54) **Device for locating the peridural space**

(30) Priority: 22.11.2001 IT FI20010220
(71) Applicant: ME.TEC. S.R.L, 52027 San Giovanni Valdarno (IT)
(72) Inventor: Ragusi, Antonino c/o ME.TEC.S.r.l., 52022 Cavriglia (IT)
(74) Representative: Gervasi, Gemma, Dr.

(57) **Abstract**

Hydroidentifier of the spinal marrow peridural space consisting of hollow body 10 and three tubes 12, 13 and 14 leading off from said hollow body 10 - out of which tubes 12 and 13 are arranged axially and tube 14 is perpendicular to the axis formed by tubes 12 and 13 - interconnected by a two-way cock housed in hollow body 10.

## Description

### Field of the invention

The present invention refers to an instrument for use in surgery, for example an identifier of the peridural space, allowing the search for and identification of the spinal marrow peridural space in an extremely precise and easy way.

### Prior art

The identification of the spinal marrow peridural space, i.e. the space before the dura mater (which essentially consists of fat and blood vessels) is mandatory e.g. when anaesthesias for surgical operations, pain-killing infiltrations, and other surgical or, more simply, therapeutic or diagnostic treatments are required.

The various means for the identification of the spinal marrow peridural space known so far exploit the difference between the peridural space pressure and the atmospheric pressure, the former being lower than the latter.

Among the aforesaid means, the following may be mentioned: Odom's indicator, Zelenka's indicator, Sagamaga's sound amplifier, Lund's test, and the more recent hanging drop technique (Gutierrez) and the loss of resistance technique (Dogliotti-Sicardi).

All aforesaid methods, which have been and are still widely used, have various drawbacks that impair the methods reliability or require - for the correct performance of the peridural space search - highly skilled personnel, endowed with remarkable deftness.

Therefore, the need for a reliable and easy-to-use instrument making up for the aforesaid cons and securing the utmost safety to operators and patients is deeply felt.

### Summary of the invention

The aforesaid problems may be overcome thanks to the spiral marrow peridural space hydroidentifier claimed in the principal claim attached hereto.

Further features of said instrument are claimed in the secondary claims.

### Description of the drawings

Figure 1 is an exploded partial view of the hydroidentifier of the invention and of the syringe and needle to be connected thereto.
Figure 2 shows a preferred embodiment of the claimed hydroidentifier.

### Detailed description of the invention

In a preferred embodiment of the invention, the spinal marrow peridural space hydroidentifier comprises hollow body 10 housing a liquid-tight two-way cock 11.

From said hollow body 10, three tubes (12, 13 and 14) lead off:
- tubes 12 and 13 are arranged along their central axis on opposite points of the surface of the hollow body 10;
- tube 14 is disposed perpendicularly to the axis formed by tubes 12 and 13.

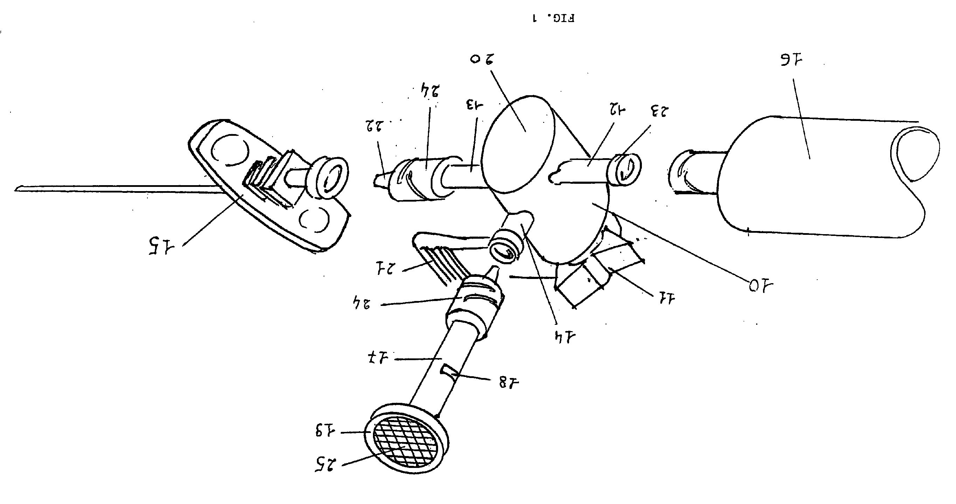

The ends opposite to the ends inserted into hollow body 10 of tubes 12, 13 and 14 are suitably shaped to be coupled with the items necessary for the identification of the spinal marrow peridural space and for the performance of the necessary operations. In Figure 1, for example, said items are represented by a needle 15 and a syringe 16.

The tube 14 (perpendicular to tubes 12 and 13) is coupled with a tube 17 provided with a cap 19 which allows the passage of air. The tube 17 can present on its surface a sight 18.

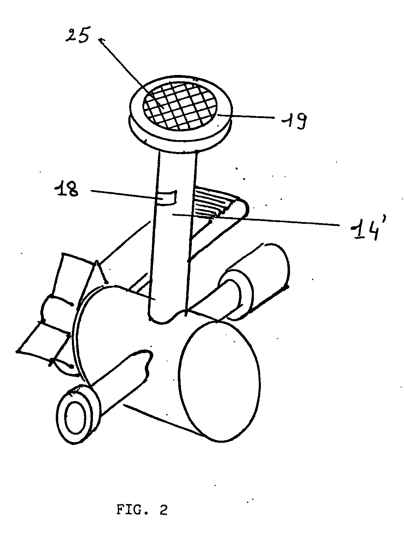

In a preferred embodiment of the invention, as illustrated in Figure 2, tubes 14 and 17 are fused in a single tube (14'), integral with the hollow body 10 and capped by cap 19.

The hollow body 10 is preferably a right cylinder presenting only one base 20, therefore it forms a chamber open on one side, wherein the liquid-tight two-way cock 11 can be housed. Altematively, even the base 20 can be absent and in such case the hollow body 10 will be a cylindrical ring wherein the liquid-tight two-way cock is inserted.

The liquid-tight two-way cock 11 may be provided with an extension 21 in order to make easier its use for the operator.

As already said, the shapes of the ends of tubes 12, 13, and 14 depend on the shape of the endings of the items to be coupled therewith or on the shape of the terminal part of tube 17 (if present).

Some of the possible shapes are:
- tapered shape 22, e.g. for fitting a needle;
- collet shape 23 e.g. for fitting a syringe by screwing;
- bayonet coupling (not shown in the Figure).

If so preferred, to secure the best seal of the items or of tube 17 (if present), fast-screwing safety couplings 24, possibly threaded, to be applied to tubes 12, 13 and 14 or to tube 17 can be envisaged.

To better guarantee the sterility of the instrument the cap 19 may be provided with an antibacterial filter 25.

The hydroidentifier according to the invention may be supplied in a sterile packet, possibly in the form of a kit separately containing the hydroidentifier itself and the other items (e.g. needle and syringe); in this case, to secure the items sterility, tubes 12 e 13 can be closed by a membrane easy to perforate. Altematively, some or all the items necessary for the necessary operations (e.g. needle and syringe) can be already assembled in their final position and the whole apparatus ready, or partially ready, for use sealed in the sterile packet

The hydroidentifier according to the invention may be made of any suitable material, preferably of transparent materials, such as glass or a plastic material.

The use of the hydroidentifier according to the invention is extremely easy.

For example, the operator inserts the needle into the cutis by means of a proper spindle according to normal practice, unscrews the spindle, and fits the hydroidentifier and the syringe to the needle.

Through the two-way cock, the operator connects tube 14 (and tube 17 coupled therewith, if present) to tube 13 and to the needle connected thereto.

After removing the cap 19 the operator fills tube 17 (and tube 14, if present), tube 13 and the needle with a suitable biocompatible liquid (possibly dyed) until the liquid reaches the sight 18 (the liquid being obviously held by the cutis in which the needle is introduced).

After performing the aforesaid steps, the operator gently pushes the whole apparatus towards the spinal marrow until that a sharp drop in the liquid level, easily detectable on the sight, makes him sure that the peridural space looked for has been reached.

At this point, by rotating the two-way cock 11, the operator connects tube 12 (and, therefore, the syringe connected thereto) to tube 13 (and, therefore, to the needle) by excluding tube 14, and injects the liquid (e.g. an anaesthetic).

If the operator accidentally pierces the dura mater, he immediately notices a rise in the liquid level above the sight 18 (in fact, the liquid contained in the hydroidentifier will be pushed back by the cephalorachidian liquid, its pressure being higher than the atmospheric pressure); therefore, he operates in safety as required by the situation without causing any involuntary and sometimes serious injury to the patient.

As may be inferred from the above description, the use of the claimed hydroidentifier of the spinal marrow peridural space secures an absolute safety and does not require particularly skilled operators; at the same time it safeguards the patient from possible involuntary injuries due to an excessive needle penetration. It follows that it makes up for all cons of the devices already known in the art.

## Claims

1. Hydroidentifier of the spinal marrow peridural space consisting of hollow body (10) housing a liquid-tight two-way cock (11) presenting three tubes (12, 13 and 14) leading off from said hollow body (10), two of such tubes, (12 and 13), being arranged along their central axis on opposite points of the surface of the hollow body (10) and the third tube (14) being perpendicular to the axis formed by tubes (12 and 13) and being capped by a cap (19) which allows the passage of the air.

2. The hydroidentifier as claimed in claim 1, wherein the tube (14) is coupled with a tube (17) provided with the cap (19).

3. The hydroidentifier as claimed in any of claims 1 and 2, wherein the hollow body (10) is a right cylinder presenting only one base (20).

4. The hydroidentifier as claimed in any of claims 1 and 2, wherein the hollow body (10) is a cylindrical ring, wherein the two-way cock (11) is inserted.

5. The hydroidentifier as claimed in any of claims 1 to 4, wherein the ends of tubes (12, 13, and 14), opposite to the ends inserted into hollow body (10), are tapered and/or collet shaped and/or shaped to allow bayonet coupling.

6. The hydroidentifier as claimed in any of claims 1 to 5, wherein tubes (12 and/or 13 and/or 14 and/or 17 (if present)) are provided with safety couplings (24) possibly threaded for fast-screwing.

7. The hydroidentifier as claimed in any of claims 1 to 6, wherein the cap (19) is provided with an antibacterial filter (25).

8. The hydroidentifier as claimed in any of claims 1 to 7 made of transparent material.

9. The hydroidenifier as claimed in claim 8, wherein said transparent material is glass or a plastic material.

10. The hydroidentifier as claimed in any of claims 1 to 9, wherein the tube (14') or the tube (17), if present, is provided with a sight (18).

11. Kit comprising the hydroidentifier as claimed in any of claims 1 to 10 and the other items required for the identification of the spinal marrow peridural space.

12. The kit as claimed in claim 11, wherein said items are a syringe and a needle for peridural injection.

13. Sterile packet containing the hydroidentifier as claimed in any of claims 1 to 10 already coupled, totally or in part, with the items required for the identification of the spinal marrow peridural space.

14. The sterile packet as claimed in claim 13, wherein said items are a syringe and a needle for peridural injection.
